(19) Europäisches Patentamt
European Patent Office
Office européen des brevets

(11) EP 1 714 973 A1

(12) **EUROPEAN PATENT APPLICATION**
published in accordance with Art. 158(3) EPC

(43) Date of publication:
**25.10.2006 Bulletin 2006/43**

(21) Application number: **04709359.6**

(22) Date of filing: **09.02.2004**

(51) Int Cl.:
***C07K 5/062*** (2006.01) ***C12P 21/02*** (2006.01)
***C12N 1/20*** (2006.01) *C12P 21/02* (2006.01)
*C12R 1/465* (2006.01) *C12N 1/20* (2006.01)
*C12R 1/465* (2006.01)

(86) International application number:
**PCT/JP2004/001311**

(87) International publication number:
**WO 2005/090384 (29.09.2005 Gazette 2005/39)**

(84) Designated Contracting States:
**CH DE FR GB LI**

(83) **Declaration under Rule 28(4) EPC (expert solution)**

(71) Applicant: **THE KITASATO INSTITUTE**
**Tokyo 108-8642 (JP)**

(72) Inventors:
- **OMURA, Satoshi**
  **Setagaya-ku, Tokyo 157-0076 (JP)**
- **TOMODA, Hiroshi**
  **Chofu-shi, Tokyo 182-0034 (JP)**
- **ABE, Akio**
  **Ohta-ku, Tokyo 144-0051 (JP)**
- **IWATSUKI, Masato**
  **Shibuya-ku, Tokyo 150-0013 (JP)**
- **TAKAHASHI, Yoko**
  **Suginami-ku, Tokyo 168-0073 (JP)**

(74) Representative: **Duckworth, Timothy John**
**J.A. Kemp & Co.,**
**14 South Square,**
**Gray's Inn**
**London WC1R 5JJ (GB)**

# (54) SUBSTANCES K01-0509 AND PROCESS FOR PRODUCING THE SAME

(57) The present invention is comprised of culturing a microorganism belonging to genus Streptomyces and having ability to produce K01-0509-A1 substance and/or K01-0509-A2 substance in a medium, accumulating K01-0509-A1 substance and/or K01-0509-A2 substance in the cultured medium and isolating K01-0509-A1 substance and/or K01-0509-A2 substance from the cultured mass. The thus obtained substances can be expected as the selective and effective pharmaceuticals for treatment of infection with enteropathogenic gram negative bacteria having type III secretion system.

EP 1 714 973 A1

**Description**

Technical Field

The present invention relates to K01-0509 substance which inhibits type III secretion mechanism of bacteria and a process for production thereof. More particularly the present invention pertains to K01-0509 substance comprising K01-0509-A1 substance and/or K01-0509-A2 substance which are useful as a remedy or preventive for infectious disease.

Background Art

The type III secretion mechanism, which is a function for releasing bacterial pathogenic factor to the extracellular field, has been reported to be highly conserved, for example, in bacteria of genus Salmonella, genus Yersinia, genus Pseudomonas, Shigella, enteropathogenic E. coli (hereinafter sometimes designates as EPEC), enterohemorrhagic E. coli and genus Bordetella (Microbiology and Molecular Biology Reviews, June, 1998, p. 381).

It has been reported that bacteria maintaining the type III secretion mechanism hereinabove released the pathogenic factor into the extracellular field through the secretion mechanism and a part of the released pathogenic factor was transferred into the host cell, and the pathogenic factor transferred into the host cell was largely involved in the pathogenicity of bacteria (Microbiology and Molecular Biology Reviews, June, 1998, p. 382 ff.).

On the other hand, it was demonstrated that EPEC strain defective in type III secretion system (hereinafter sometimes designates as type III secretion protein) lost the pathogenicity in the infectious experiments using rabbits (J. Exp. Med. 188 (10), 1907-1916, 1998, November 16) and the infectious experiment using human volunteers (Infection and Immunity, June 2000, p. 3689-3695). From these facts, substances inhibiting the type III secretion system and the function of the secretion protein are expected to exhibit effects as a remedy or preventive remedy for infectious disease having new ideas without killing bacteria but making the pathogenicity disappear.

Disclosure of the invention

In such circumstances, problem to be solved by the present invention is to find out new antiinfectious disease agents which allow to disappear the pathogenicity of pathogenic bacteria.

In order to solve such problems hereinabove described, we have continued studies on microbial metabolites, and found that substances having inhibitory activities against the type III secretion system were produced in the cultured medium of a newly isolated strain, designated as K01-0509, from soil sample collected in Amami-Oshima. Further, we have found that substances represented by the chemical structure of the formula [I] and [II] hereinbelow as a result of isolation and purification of the active principle from the cultured mass showing inhibitory activities against the type III secretion system. Since substances having such chemical structures were not known, these substances were designated as K01-0509-A1 substance and K01-0509-A2 substance, and were totally designated as K01-0509 substance.

The present invention has been completed by such knowledge.

An aspect of the present invention is to provide K01-0509-A1 substance represented by the following formula [I] ;

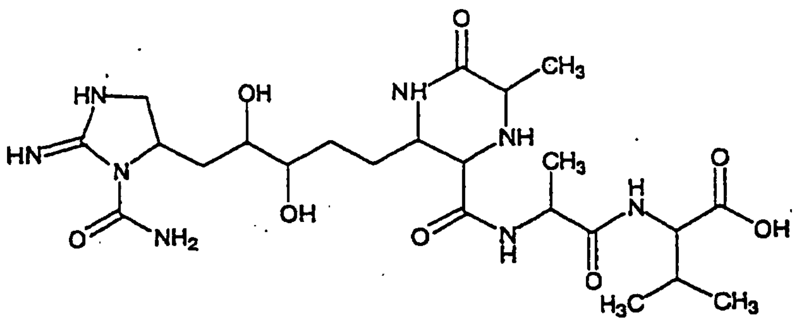

[ I ]

An aspect of the present invention is to provide K01-0509-A2 substance (a stereoisomer of A1 substance) represented by the following formula [II];

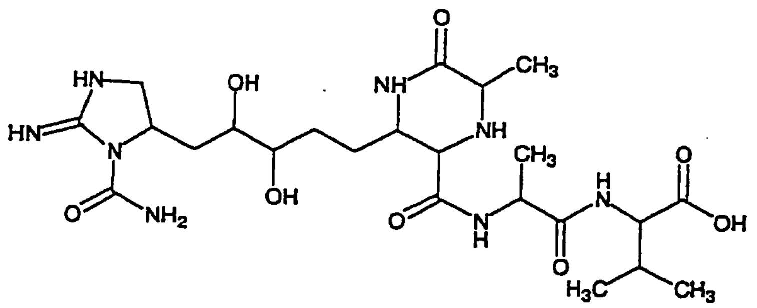

[I.I]

Further aspect of the present invention is to provide K01-0509A substance consisting of K01-0509-A1 substance represented by the following formula [I];

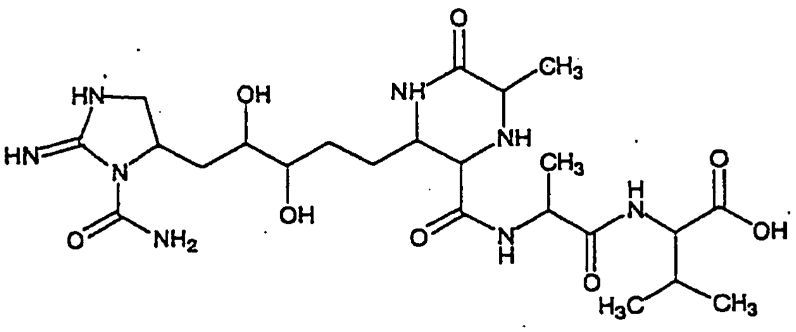

[I]

and K01-0509-A2 substance represented by the following formula [II] ;

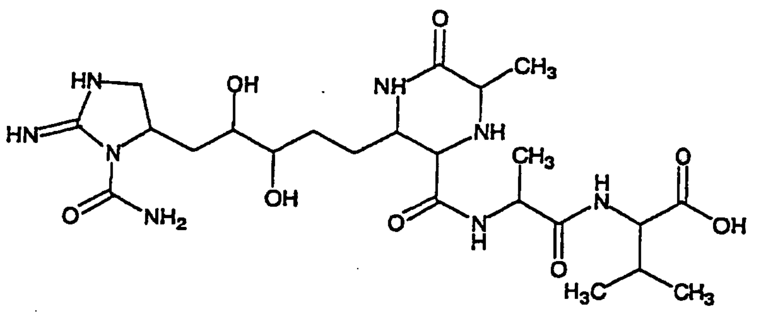

[I.I]

Another aspect of the present invention is to provide a process for production of K01-0509-A1 substance comprising culturing microorganism belonging to genus Streptomyces and having ability to produce K01-0509-A1 substance, accumulating K01-0509-A1 substance in a cultured medium and isolating K01-0509-A1 substance from the cultured mass.

More further aspect of the present invention is to provide a process for production of K01-0509-A2 substance comprising culturing microorganism belonging to genus Streptomyces and having ability to produce K01-0509-A2 substance, accumulating K01-0509-A2 substance in a cultured medium and isolating K01-0509-A2 substance from the cultured mass.

Further aspect of the present invention is to provide a process for production of a composition consisting of K01-0509-A1 substance and K01-0509-A2 substance comprising culturing microorganism belonging to genus Streptomyces and having ability to produce K01-0509-A1 substance and K01-0509-A2 substance, accumulating K01-0509-A1 substance and K01-0509-A2 substance in a cultured medium and isolating K01-0509-A1 substance and K01-0509-A2 substance from the cultured mass.

Further aspect of the present invention is to provide a process for production of K01-0509-A1 substance wherein

a microorganism belonging to genus Streptomyces and having ability to produce K99-05278-A1 substance is Streptomyces sp. K01-0509 FERM BP-08504.

Further aspect of the present invention is to provide a process for production of K01-0509-A2 substance wherein a microorganism belonging to genus Streptomyces and having ability to produce K99-05278-A2 substance is Streptomyces sp. K01-0509 FERM BP-08504.

Further aspect of the present invention is to provide a process for production of a composition consisting of K01-0509-A1 substance and K01-0509-A2 substance wherein a microorganism belonging to genus Streptomyces and having ability to produce K99-05278-A1 substance and K01-0509-A2 substance is Streptomyces sp. K01-0509 FERM BP-08504.

Further aspect of the present invention is to provide a microorganism which is Streptomyces sp. K01-0509 FERM BP-08504.

The microorganism having ability to produce K01-0509-A1 substance and K01-0509-A2 substance or a composition thereof represented by the formula [I] and [II] hereinbefore (hereinafter designates as "K01-0509 substance producing microorganism") belongs to genus Streptomyces, and, for example, Streptomyces sp. K01-0509, which was newly isolated by the present inventors, is the most preferable strain used in the present invention.

Taxonomical properties of Streptomyces sp. K01-0509 of the present invention are as follows.

(I) Morphological properties

Vegetative mycelia grow well on various agar media and no fragmentation is observed. Aerial mycelia are abundantly grown on yeast-malt extract agar medium and glycerol-asparagine agar medium, and exhibit white to grayish color. On microscopic observation, chains of more than 20 spores are observed on the aerial mycelia, and the morphological form is linear chains and size of spore is about 0. 6 - 0.8 × 1.0 - 1.8 μm with cylindrical form. Surface of the spore is smooth. Sclerotia, sporangia and zoospores are not observed.

(II) Culture properties on various media

Culture properties of the producing strain of the present invention determined by the method of E. B Shirling and D. Gottlieb (International Journal of Systematic Bacteriology, 16: 313, 1966) are shown in the following. Color tone was determined referring to Color Harmony Manual, 4th Ed. (Container Corporation of America, Chicago, 1958) as a standard color, and color name as well as attached code number in the parenthesis. Unless otherwise noted, results are observation of cultures at 27°C for 2 weeks on various media.

| Culture properties | |
|---|---|
| Sucrose-nitrate agar medium | |
| Growth | good growth, light amber (3ic) |
| Reverse side | light amber (3ic) |
| Aerial mycelium | moderate epiphytic, white (a) - gray (h) |
| Soluble pigment | none |
| Glucose-asparagine agar medium | |
| Growth | good growth, pearl pink (3ca) |
| Reverse side | pearl pink (3ca) |
| Aerial mycelium | none |
| Soluble pigment | none |
| Glycerol-asparagine agar medium (ISP) | |
| Growth | good growth, light amber (3ic) |
| Reverse side | light amber (3ic) |
| Aerial mycelium | abundantly epiphytic, white (a) - gray (f) |
| Soluble pigment | none |
| Starch-inorganic salt agar medium (ISP) | |
| Growth | good growth, light amber (3ic) |

(continued)

| | |
|---|---|
| Starch-inorganic salt agar medium (ISP) | |
| Reverse side | light tan (3gc) - light amber (3ic) |
| Aerial mycelium | moderate epiphytic, white (a) - gray (h) |
| Soluble pigment | none |
| Tyrosine agar medium (ISP) | |
| Growth | good growth, bamboo (2gc) |
| Reverse side | bamboo (2gc) |
| Aerial mycelium | moderately epiphytic, white (a) |
| Soluble pigment | none |
| Oatmeal agar medium (ISP) | |
| Growth | good growth, light amber (3ic) |
| Reverse side | light tan (3gc) - orange rust (4pe) |
| Aerialmycelium | moderately epiphytic, white (a) - dark cobalt gray (2ih) |
| Soluble pigment | none |
| Yeast-malt extract agar medium (ISP) | |
| Growth | good growth, amber (31c) |
| Reverse side | light amber (3ic) - dark luggage tan (4pg) |
| Aerial mycelium | abundantly epiphytic, white (a)- gray (g) |
| Soluble pigment | none |
| Nutrient agar medium | |
| Growth | good growth, pearl pink (3ca) |
| Reverse side | pearl pink (3ca) |
| Aerial mycelium | none |
| Soluble pigment | none |
| Peptone-yeast-iron agar medium (ISP) | |
| Growth | good growth, bamboo (2fb) |
| Reverse side | bamboo (2fb) |
| Aerial mycelium | poorly epiphytic, light ivory (2ca) |
| Soluble pigment | none |
| Glucose-nitrate agar medium | |
| Growth | moderate growth, bright (3ia) |
| Reverse side | orange (41a) |
| Aerial mycelium | none |
| Soluble pigment | none |
| Glycerol-calcium malate agar medium | |
| Growth | good growth, bamboo (2fb) |
| Reverse side | pearl pink (3ca) |
| Aerial mycelium | poorly epiphytic, white (a) |
| Soluble pigment | none |

(continued)

| | |
|---|---|
| Glucose-peptone agar medium | |
| Growth | good growth, light ivory (2ca) |
| Reverse side | pearl pink (3ca) |
| Aerial mycelium | none |
| Soluble pigment | none |

(III) Physiological properties

| | | | |
|---|---|---|---|
| (1) | Formation of melanin pigment | | |
| | (a) Tyrosine agar | | negative |
| | (b) Peptone-yeast-iron agar medium | | negative |
| | (c) Tryptone-yeast liquid | | positive |
| | (d) Simple gelatin medium (21 - 23°C) | | false positive |
| (2) | Nitrate reduction | | negative |
| (3) | Liquefaction of gelatin (21 - 23°C) (simple gelatin medium) | | negative |
| (4) | Starch hydrolysis | | positive |
| (5) | Coagulation of defatted milk (37°C) | | positive |
| (6) | Peptonization of defatted milk (37°C) | | positive |
| (7) | Growth temperature | | 10 - 38°C |
| (8) | Utilization of carbon sources (Pridham-Gottlieb agar medium) | | |
| | Utilize: | D-glucose, L-arabinose | |
| | Not utilize: | D-xylose, D-mannitol, L-rhamnose, D-fructose, myo-inositol, raffinose, melibiose, sucrose | |
| (9) | Decomposition of cellulose | | negative |

(IV) Composition of cell wall

2,6-diaminopimelic acid of cell wall is LL type. Main menaquinone is MK-9($H_6$) and MK-9 ($H_8$).

(V) Conclusion

Taxonomical properties of the strain of the present invention are summarized as follows. 2,6-diaminopimelic acid in the cell wall is LL type and main menaquinone is MK-9 ($H_6$) and MK-9 ($H_8$). Morphology of the spore chain is linear chain, forming with long spore chains and smooth spore surface. Various properties on the culture are exhibiting brown color tone vegetative mycelia and white to grayish aerial mycelia. Production of melanin pigment is observed in Tryptone-yeast liquid.

According to results hereinabove, the present strain was identified as the strain belonging to genus Streptomyces based on descriptions in "Bergey' s Manual of Systematic Bacteriology, Vol. 4, 1989".

The strain was deposited, based on Budapest Treaty on the International Recognition of the Deposit of Microorganisms for the Purposes of Patent Procedure, as Streptomyces sp. K01-0509 in International Patent Organism Depository, National Institute of Advanced Industrial Science and Technology, AIST Tsukuba Central 6, 1-1, Higashi 1-chome, Tsukuba-shi, Ibaraki-ken, 305-8566 Japan on October 6, 2003 as permanent depository number FERM BP-08504.

The strain of Streptomyces sp. K01-0509 can be mentioned as a preferable example of K05-0509 substance producing strain used in the present invention. However, since the morphological properties of microorganisms are generally very easily mutated and are not constant. Natural mutation or artificial mutation generally performed by ultraviolet irradiation or chemical mutagens such as N-methyl-N'-nitro-N-nitrosoguanidine and ethyl methansulfonate, are well known. The strain belonging to genus Streptomyces and having ability to produce K01-0509 substance represented by the formula [I] and [II] hereinbefore, including the artificial mutants as well as natural mutants or composition thereof, can be used in the present invention.

In a production of K01-0509 substance of the present invention, at first, K01-0509 substance producing strain belonging to genus Streptomyces is cultured in a preferable medium. Nutrient sources preferable for production of K01-0509 substance of the present invention are assimilable carbon sources for microorganism, digestible nitrogen sources and, if necessary, inorganic salts. Examples of assimilable carbon sources are sugars such as glucose, fructose, maltose, lactose, galactose, dextrin and starch, and plant oil such as soybean oil, etc. are used independently or in combination.

Examples of nitrogen sources are peptone, yeast extract, meat extract, soybean powder, cotton seed powder, corn steep liquor, malt extract, casein, amino acids, urea, ammonium salts and nitrates are used independently or in combination. If necessary, salts such as phosphate, magnesium, calcium, sodium, potassium, heavy metallic salts such as iron, manganese, copper, cobalt or zinc, vitamins and substances suitable for production of K01-0509 substance are added.

In the liquid culture, if foaming occurs, antifoam agents such as liquid paraffin, animal oil, vegetable oil, silicone oil and surface active agent can preferably be added. The above culture can be performed by liquid or solid culture condition, if the above nutrient sources are contained, and in general, the culture can preferably be performed using liquid culture medium, and in case of small production, the culture using flask is preferable.

In the large scale production using the large tank, in order to prevent delay of growth of microorganism in the production process, the production strain is inoculated and cultured initially in relatively small amount of culture medium, subsequently the cultured mass is transferred into the large tank and cultivation is preferably continued. In this case, compositions of the medium used in the pre-culture and the medium used in the production culture can be identical or different if necessary.

In the culture under aeration spinning condition, conventional means, for example, agitation using propeller and other mechanical stirring, rotation or shaking in fermenter, treating with pumping and blowing air can be applied. Air for aeration should be sterilized. Culturing temperature can be applied within ranges in the production of K01-0509 substance by K01-0509 substance producing strain, and the cultivation is performed usually at 20 - 30°C, preferably at 27°C. Culturing pH is usually pH 5 - 8, preferably about pH 7. Culturing time depends on culturing condition and is usually for 3 days. The thus obtained accumulated K01-0509 substance in the cultured mass exists generally in cultured supernatant. Isolation of K01-0509 substance from the cultured medium can be performed by methods used for isolation of metabolites from microbial cultured mass independently, repeatedly or in combination with any orders of the means.

Isolation and collection of K01-0509 substance can be performed by collecting from the cultured supernatant. The cultured supernatant is treated by the known method used for collection of water soluble substance, for example, chromatography such as adsorption chromatography, gel filtration chromatography and high performance liquid chromatography, in combination or repetition thereof to isolate K01-0509 substance.

Physicochemical properties of K01-0509 substance of the present invention are explained hereinbelow.

1. KO1-0509-A1 substance

(1) Nature: white powder
(2) Molecular formula: $C_{23}H_{40}N_8O_8$
HRFAB-MS(m/z) $[M+H]^+$
Calculated 557.3047, Found 557.3052
(3) Molecular weight: 556
FAB-MS(m/z)$[M+H]^+$ 557, $[M+Na]^+$ 579
(4) Ultraviolet absorption spectrum (in water) : as shown in Fig. 1, terminal absorption
(5) Infrared absorption spectrum (KBr Tablet) : as shown in Fig. 2, specific maximum absorption $\lambda$max at 1564, 1682 $cm^{-1}$.
(6) Specific rotation: $-[\alpha]_D^{27}$= -5.00° (c= 0.1, methanol)
(7) Solubility in solvent: soluble in water, dimethyl sulfoxide (DMSO) and methanol,
: insoluble in acetonitrile, ethyl acetate, chloroform and acetone.
(8) Grouping for acidic, neutral and basic: Basic substance.
(9) Amino acid analysis: L-alanine and L-valine (1 : 1).
(10) $^1$H nuclear magnetic resonance spectrum (in deuterium oxide) measured by using Varian NMR 400 MHz (Fig. 3). Chemical shifts of hydrogen (ppm) are: 0.92(3H), 0.92(3H), 1.39(3H), 1.44, 1.55(3H), 1.57, 1.71, 1.76, 1.77, 1.93, 2.11, 3.48, 3.61, 3.79, 3.97, 4.07, 4.17, 4.23, 4.27, 4.43 and 4.44.
(11) $^{13}$C-nuclear magnetic resonance spectrum (in deuterium oxide) measured by using Varian NMR 100 MHz (Fig. 4). Chemical shifts of carbon (ppm) are: 18.2, 19.1, 20.1, 21.1, 29.6, 30.3, 32.5, 38.8, 52.2, 53.1, 53.3, 53.8, 54.0, 56.0, 62.4, 74.1, 76.7, 158.9, 158.9, 168.4, 172.1, 176.6 and 179.2.

As shown in above, as a result of detailed examination of various physico-chemical properties and spectral data of K01-0509-A1 substance, K01-0509-A1 substance was determined to have the chemical structure as shown in the formula [I].

2. K01-0509-A2 substance

(1) Nature: white powder
(2) Molecular formula: $C_{23}H_{40}N_8O_8$
HRFAB-MS(m/z)$[M+H]^+$
Calculated 557.3047, Found 557.3023
(3) Molecular weight: 556
FAB-MS(m/z) $[M+H]^+$ 557, $[M+Na]^+$ 579
(4) Ultraviolet absorption spectrum (in water) : as shown in Fig. 5, terminal absorption
(5) Infrared absorption spectrum (KBr Tablet) : as shown in Fig. 6, specific maximum absorption λmax at 1564, 1682 $cm^{-1}$.
(6) Specific rotation: $[\alpha]_D^{26}$= -7.4° (c= 0.1, methanol)
(7) Solubility in solvent: soluble in water, dimethyl sulfoxide (DMSO) and methanol,
: insoluble in acetonitrile, ethyl acetate, chloroform and acetone.
(8) Grouping for acidic, neutral and basic: Basic substance.
(9) Amino acid analysis: L-alanine and L-valine (1 : 1).
(10) $^1H$ nuclear magnetic resonance spectrum (in deuterium oxide) measured by using Varian NMR 400 MHz (Fig. 7). Chemical shifts (ppm) are: 0.92(3H), 0.92(3H), 1.39(3H), 1.44, 1.53(3H), 1.57, 1.71, 1.76, 1.77, 1.93, 2.12, 3.48, 3.61, 3.79, 3.99, 4.07, 4.09, 4.23, 4.27, 4.44 and 4.50.
(11) $^{13}C$-nuclear magnetic resonance spectrum (in deuterium oxide) measured by using Varian NMR 100 MHz (Fig. 8). Chemical shifts (ppm) are: 17.0, 19.2, 20.1, 21.1, 30.1, 30.4, 32.6, 38.8, 52.2, 53.2, 53.8, 54.0, 54.9, 59.8, 62.4, 74.2, 76.8, 158.9, 159.9, 168.3, 172.1, 176.6 and 179.0.

As shown in above, as a result of detailed examination of various physico-chemical properties and spectral data of K01-0509-A2 substance, K01-0509-A2 substance was determined to have the chemical structure as shown in the formula [II].

Biological properties of K01-0509 substance of the present invention are explained in detail hereinbelow. Inhibitory activity against the type III secretion system and antibacterial activity were assayed by the following methods.

(1) Assay of enteropathogenic E. coli type III secretion system dependent hemolytic inhibitory activity

Test was performed according to method for detecting substances inhibiting the bacterial type III secretion system and function of secretory proteins thereof which was established by Omura et al. (WO 02/057760A1 and corresponding US Patent 6, 586, 200) . One loopful enteropathogenic E. coli cesT defective strain (WO 02/057760A1) was inoculated in LB liquid medium (EB media 2.5%, Funakoshi Co. Ltd., Japan) (5 ml) and cultured at 37°C for 12 hours without shaking. Bacterial culture (1 %) was inoculated to M9 medium containing casamino acid (sodium dihydrogen phosphate 0.68% (Kanto Chemical Inc., Japan), potassium dihydrogen phosphate 0.3% (Wako Pure Chemical Industries Ltd., Japan), sodium chloride 0.05% (Kanto Chemical Inc., Japan), ammonium chloride 0.1% (Wako Pure Chemical Industries Ltd., Japan), glucose 0.4% (Wako Pure Chemical Industries Ltd., Japan), casamino acid 0.1% (Sanko Junyaku Co., Ltd., Japan) and magnesium sulfate 0.012% (Kanto Chemical Inc., Japan)) and cultured at 37°C for further 4 hours without shaking. Cultured liquid was centrifuged at 3500 rpm for 15 minutes to collect bacterial cell precipitation. The bacterial pellets were suspended in the fresh M9 medium (5 ml) to prepare the test bacterial liquid.

Erythrocytes was washed three times by centrifugation of the erythrocytes suspension, which was prepared by adding physiological saline (40 ml) to the sheep red blood cells (8 ml) (obtainable from Nippon Biological Materials Center, Japan), at 4°C at 2500 rpm for 5 minutes. Weight of the erythrocyte pellets was measured. M9 medium containing casamino acid (2 ml) was added to the erythrocytes pellet (1 g) by a ratio thereof to prepare suspension of erythrocytes. The thus prepared E. coli suspension and erythrocyte suspension were mixed in equal quantities. The mixture (90 μl) was added to the 96-well microplate (Corning Inc., U.S.A.), to which the sample (5 μl) and M9 medium containing casamino acid (10 μl) were previously added. The plate was centrifuged at 1500 rpm for 10 minutes, and the hemolytic reaction was initiated at 37°C for 90 - 150 minutes. After the reaction, cooled PBS(-) (150 μl)(sodium chloride 0.8% (Kanto Chemical Inc., Japan), sodium dihydrogen phosphate 0.115% (Kanto Chemical Inc., Japan), potassium dihydrogen phosphate 0.02% (Wako Pure Chemical Industries Ltd., Japan) and potassium chloride 0.02% (Kanto Chemical

Inc., Japan)) was added to prepare the suspension, and was centrifuged at 1500 rpm for 10 minutes. Supernatant (100 μl) obtained by the centrifugation was transferred into another 96-well microplate (Corning Inc., U.S.A.). Eluted hemoglobin was measured at 550 nm by using automatic microplate reader (Bio-Instruments Inc., U.S.A.). The hemolytic inhibitory activity is calculated by the following equation.

$$\text{Inhibition rate (\%)} = 100 - [(A-C)/(B-C)\text{.times.}100]$$

wherein

A: data at 550 nm when added the sample
B: data at 550 nm of the mixture of erythrocyte and enteropathogenic E.coli CesT defective strain alone
C: data at 550 nm of erythrocytes alone Results are as follows.

The drug concentration causing 50 % hemolytic inhibitory activity ($IC_{50}$) for K01-0509-A1 substance is 4.2 μg/ml and $IC_{50}$ for K01-0509-A2 substance is 3.2 μg/ml. Both substances were demonstrated to inhibit type III secretion system.

(2) Assays of antibiotic activities for enteropathogenic E. coli and various test microorganisms by paper disc method

Test microorganisms used were enteropathogenic E. coli E2348/69 (wild strain), Bacillus subtilis ATCC 6633, Micrococcus luteus ATCC 9341, E. coli NIHJ, and Xanthomonas campestris pv. oryzae KB88. Medium used was nutrient agar medium (peptone 0.5% (Kyokuto Pharmaceutical Industrial Co., Ltd. , Japan), meat extract 0.5% (Kyokuto Pharmaceutical Industrial Co., Ltd., Japan) and agar 0.8% (Shimizu Shokuhin K.K., Japan), adjusted at pH 7.0). Activity was evaluated by paper disc method (diameter 6 mm: Advantech Co. Ltd.), and inhibitory zone was measured after 24 hours cultivation.

As a result, no inhibition zone was shown in all test microorganisms for K01-0509-A1 at 10 μg/disc and K01-0509-A2 at 10 μg/disc.

As described in detail, K01-0509 substance of the present invention can be expected as novel anti-infectious disease drug, which inhibits selectively type III secretion system without exhibiting antibacterial activity.

Brief explanation of drawings

Fig. 1 shows ultraviolet absorption spectrum of K01-0509-A1 substance (in water).
Fig. 2 shows infrared absorption spectrum of K01-0509-A1 substance (KBr tablet).
Fig. 3 shows proton nuclear magnetic resonance spectrum of K01-0509-A1 substance (in deuterium oxide).
Fig. 4 shows carbon nuclear magnetic resonance spectrum of K01-0509-A1 substance (in deuterium oxide).
Fig. 5 shows ultraviolet absorption spectrum of K01-0509-A2 substance (in water).
Fig. 6 shows infrared absorption spectrum of K01-0509-A2 substance (KBr tablet).
Fig. 7 shows proton nuclear magnetic resonance spectrum of K01-0509-A2 substance (in deuterium oxide).
Fig. 8 shows carbon nuclear magnetic resonance spectrum of K01-0509-A2 substance (in deuterium oxide).

Best mode for carrying out the invention

The present invention will be explained by illustrating example, but the present invention is not limited within the example.

Example

A loopful of the strain K01-0509 cultured by an agar slant medium (starch 1.0% (Wako Pure Chemical Industries Ltd., Japan), N-Z amine 0.3% (Wako Pure Chemical Industries Ltd., Japan), meat extract 0.1% (Kyokuto Pharmaceutical Industrial Co., Ltd., Japan), $CaCO_3$ 0.3% (Kanto Chemical Inc., Japan) and agar 1.2% (Shimizu Shokuhin K.K., Japan), adjusted at pH 7.0) was inoculated into a medium (100ml) (starch 2.4% (Wako Pure Chemical Industries Ltd., Japan), glucose 0.1% (Wako Pure Chemical Industries Ltd., Japan), peptone 0.3% (Kyokuto Pharmaceutical Industrial Co., Ltd., Japan), yeast extract 0.5% (Oriental Yeast Co., Ltd., Japan) and $CaCO_3$ (Kanto Chemical Inc., Japan), adjusted at pH

7.0) in a 500-ml Erlenmeyer flask and cultured at 27°C for 3 days by using rotary shaker (210 rpm) to obtain seed culture liquid. The seed culture (200 ml) was inoculated into a production medium (20 liters) (starch 2.4% (Wako Pure Chemical Industries Ltd., Japan), glucose 0.1% (Wako Pure Chemical Industries Ltd., Japan), peptone 0.3% (Kyokuto Pharmaceutical Industrial Co., Ltd., Japan), meat extract 0.3% (Kyokuto Pharmaceutical Industrial Co., Ltd., Japan), yeast extract 0.5% (Oriental Yeast Co., Ltd., Japan), calcium carbonate 0.4% (Kanto Chemical Inc., Japan), iron sulfate 7 hydrate $5.0 \times 10^{-4}$% (Kanto Chemical Inc., Japan), magnesium chloride 4 hydrate $5.0 \times 10^{-4}$% (Wako Pure Chemical Industries Ltd., Japan), copper sulfate 5 hydrate $5.0 \times 10^{-4}$% (Kanto Chemical Inc., Japan) and cobalt chloride 6 hydrate $5.0 \times 10^{-4}$% (Wako Pure Chemical Industries Ltd., Japan)) in a 30-L jar-fermenter, and cultured at 37°C for 4 days.

The medium cultured for 4 days (a total of 54 liters) was centrifuged by using Sharpless centrifuge to separate into the supernatant and microbial cells. The supernatant was charged on a column of active carbon (φ75 × 150 mm, Wako Pure Chemical Industries Ltd., Japan), washed with water (1.5 liter), eluted the active principle with 20 and 40% acetone (each 1.5 liter), concentrated in vacuo and lyophilized. The obtained crude substance (21.5 g) dissolved in a small amount of water was charged on a column of Amberlite IRC-50 (H+) (φ46 × 110 mm, Organo Corp., Japan), washed with water (300 ml). The active principle was eluted with 1N HCl (600 ml), neutralized, desalted by means of electrodialysis, concentrated in vacuo and lyophilized to obtain crude extract (823 mg).

The crude extract (200 mg) was dissolved in a small amount of water, and charged on an ODS column (φ10 × 30 mm, Senshu Scientific Co., Ltd., Japan) equilibrated with 0.1% aqueous trifluoroacetic acid solution, washed with 0.1% aqueous trifluoroacetic acid solution (10 ml), eluted the active principle with 20% methanol in 0.1% aqueous trifluoroacetic acid solution (5 ml), concentrated in vacuo and lyophilized. The remained crude substance (623 mg) was treated by the same manner to obtain active substance (103 mg). The active substance (103 mg) was dissolved in a small amount of water, and was purified by using the preparative HPLC (column: Develosil C30-UG-5, φ20 × 250 mm, Nomura Chemical Co., Ltd., Japan). The UV absorption at 210 nm was monitored in the isocratic mobile phase of 8% methanol in 0.1% aqueous trifluoroacetic acid solution at flow rate of 5 ml/min. A peak showing activity at the retention time of 88 min. was observed and collected. The collected solution was concentrated in vacuo and lyophilized.

The obtained active material (8.3 mg) was dissolved in a small amount of water and purified by using the preparative HPLC (column: Develosil C30-UG-5, φ20 × 250 mm, Nomura Chemical Co., Ltd., Japan). The UV absorption at 210 nm was monitored in the isocratic mobile phase of 3% acetonitrile in 0.05% aqueous phosphoric acid solution at flow rate of 5 ml/min. Peaks showing activity at the retention times of 27 min. and 30 min. were observed and collected. The collected solution was concentrated in vacuo, and charged on the ODS column (φ5 × 10 mm, Senshu Scientific Co., Ltd., Japan) equilibrated with 0.1% aqueous trifluoroacetic acid solution, washed with 0.1% aqueous trifluoroacetic acid solution (5 ml), eluted the active substance with 100% methanol with 0.1% (final concentration) trifluoroacetic acid (5 ml) concentrated in vacuo and lyophilized to obtain white powdery K01-0509-A1 substance (1.1 mg) and K01-0509-A2 substance (1.5 mg).

Industrial applicability

As explained hereinabove, the microorganism represented by the strain K01-0509, having ability to produce K01-0509-A1 substance and K01-0509-A2 substance, belonging to genus Streptomyces is cultured in a medium, and K01-0509-A1 substance and K01-0509-A2 substance having inhibitory activity against type III secretion system are isolated. These substances can be expected as the selective and effective pharmaceuticals for treatment or prevention of infection with enteropathogenic gram negative bacteria having type III secretion system.

## Claims

1. K01-0509-A1 substance represented by the formula [I];

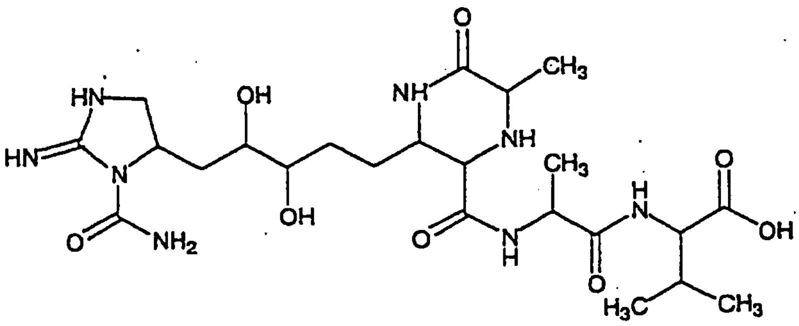

[I]

2. K01-0509-A2 substance represented by the formula [II];

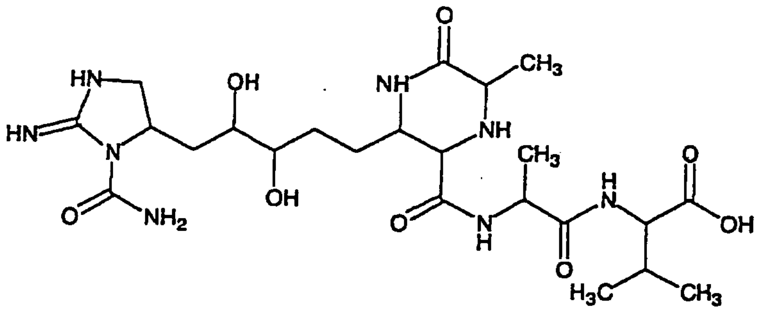

[II]

which is a stereo isomer of K01-0509-A1 substance.

3. A composition of K01-0509 substance consisting of specifically K01-0509-A1 substance represented by the formula [I];

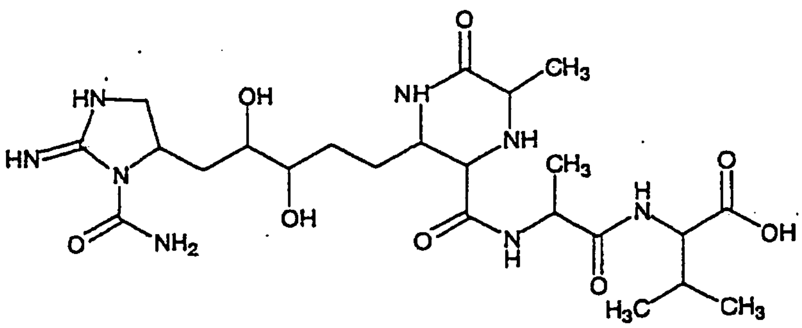

[I]

and/or
specifically K01-0509-A2 substance represented by the formula [II];

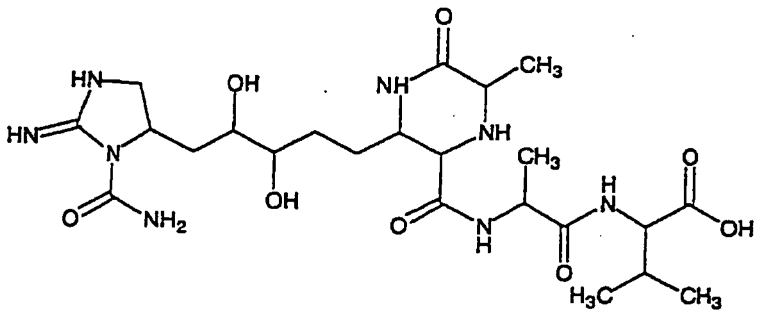

[I.I]

which is a stereo isomer of K01-0509-A1 substance.

4. A process for production of K01-0509-A1 substance, which is described in claim 1, comprising culturing a microorganism belonging to genus Streptomyces and having ability to produce K01-0509-A1 substance in a medium, accumulating K01-0509-A1 substance in the cultured medium and isolating K01-0509-A1 substance from the cultured mass.

5. A process for production of K01-0509-A2 substance, which is described in claim 2, comprising culturing a microorganism belonging to genus Streptomyces and having ability to produce K01-0509-A2 substance in a medium, accumulating K01-0509-A2 substance in the cultured medium and isolating K01-0509-A2 substance from the cultured mass.

6. A process for production of the composition, which is described in claim 3, comprising culturing a microorganism belonging to genus Streptomyces and having ability to produce K01-0509-A1 substance and/or K01-0509-A2 substance in a medium, accumulating K01-0509-A1 substance and/or K01-0509-A2 substance, in the cultured medium and isolating K01-0509-A1 substance and/or K01-0509-A2 substance from the cultured mass.

7. The process for production of K01-0509-A1 substance according to claim 4 wherein the microorganism belonging to genus Streptomyces and having ability to produce K01-0509-A1 substance is Streptomyces sp. K01-0509 FERM BP-08504.

8. The process for production of K01-0509-A2 substance according to claim 5 wherein the microorganism belonging to genus Streptomyces and having ability to produce K01-0509-A2 substance is Streptomyces sp. K01-0509 FERM BP-08504.

9. The process for production of the composition consisting of and/or K01-0509-A2 substance according to claim 6 wherein the microorganism belonging to genus Streptomyces and having ability to produce K01-0509-A1 substance and/or K01-0509-A2 substance is Streptomyces sp. K01-0509 FERM BP-08504.

10. A microorganism Streptomyces sp. K01-0509 FERM BP-08504.

11. The microorganism according to claim 10 wherein the microorganism is mutant of Streptomyces sp. K01-0509 FERM BP-08504.

FIG. 1

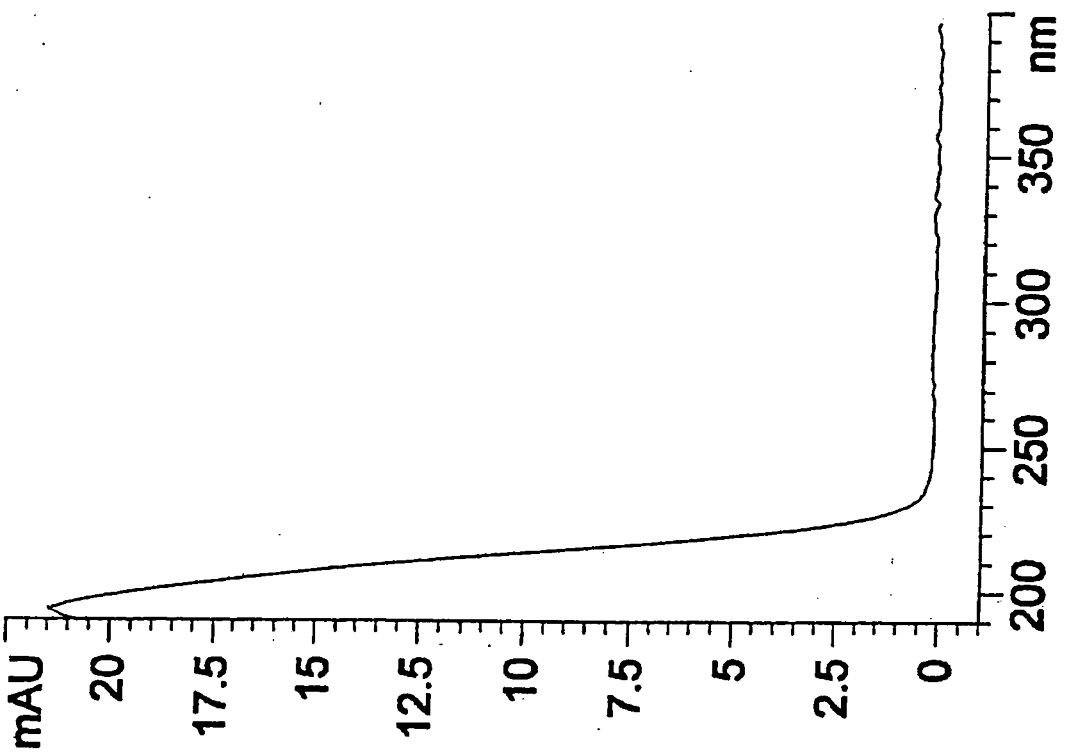

FIG. 2

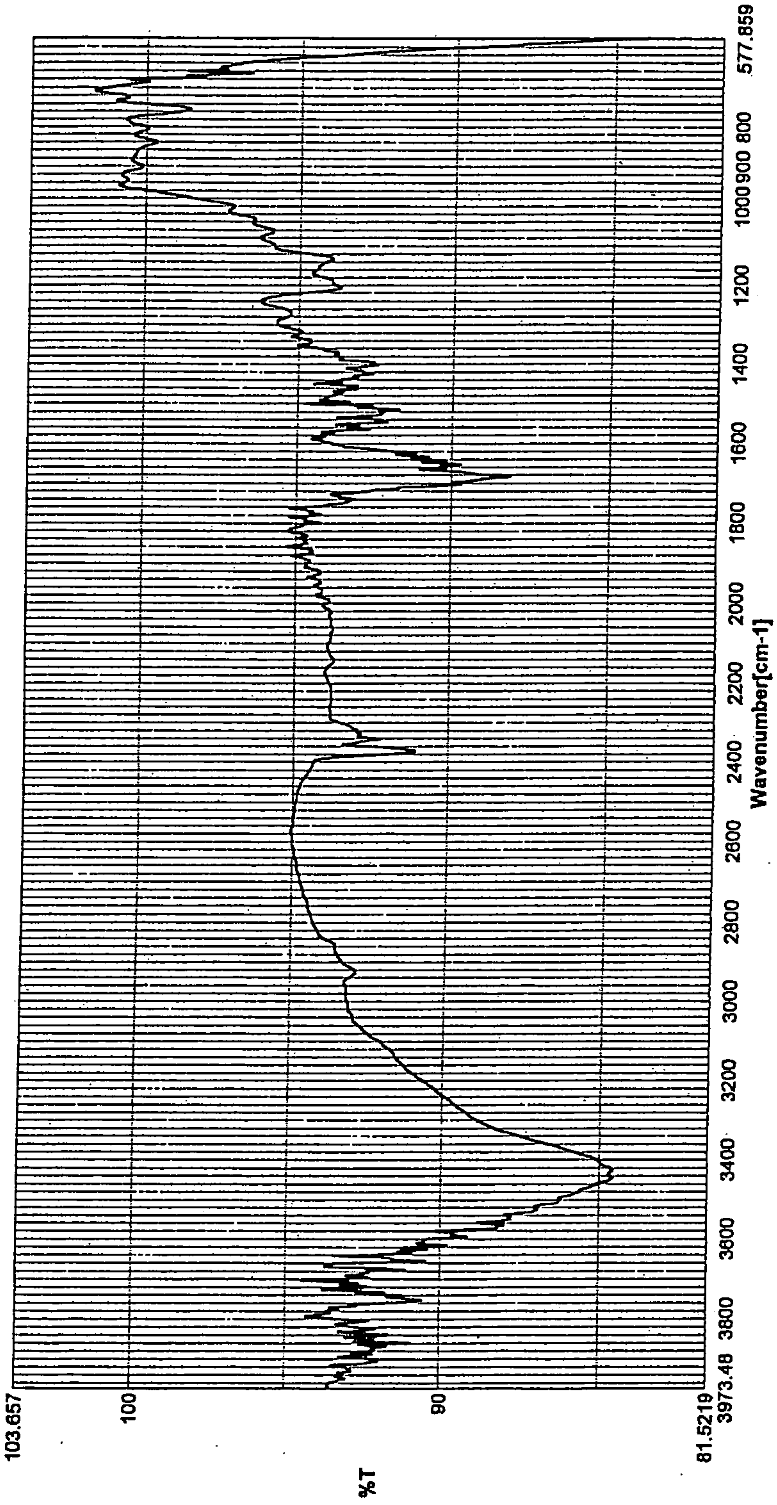
103.657
100
90
81.5219
%T
3973.48
3800
3600
3400
3200
3000
2800
2600
2400
2200
2000
1800
1600
1400
1200
1000
900
800
577.859
Wavenumber[cm-1]

FIG. 3

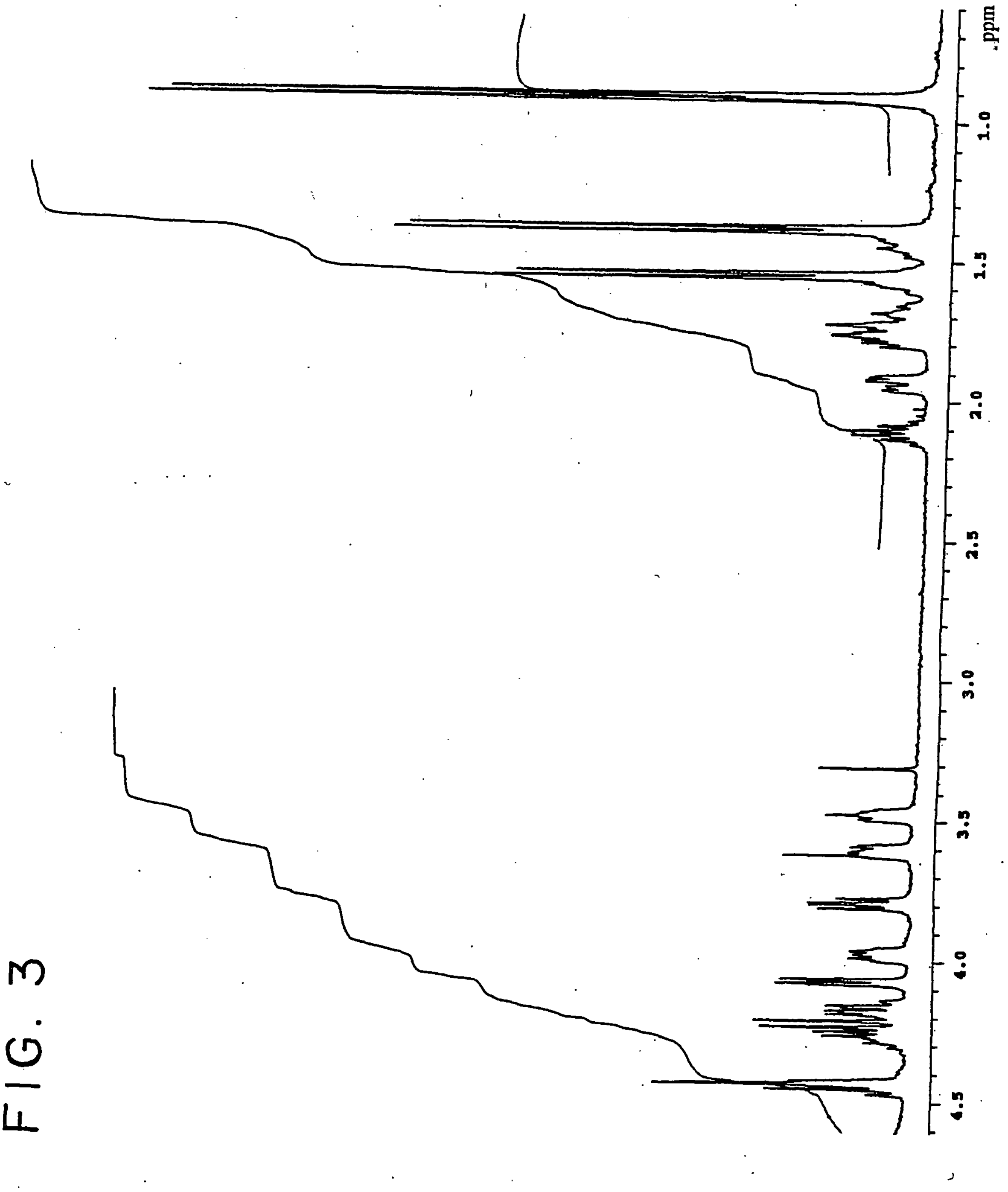

FIG. 4

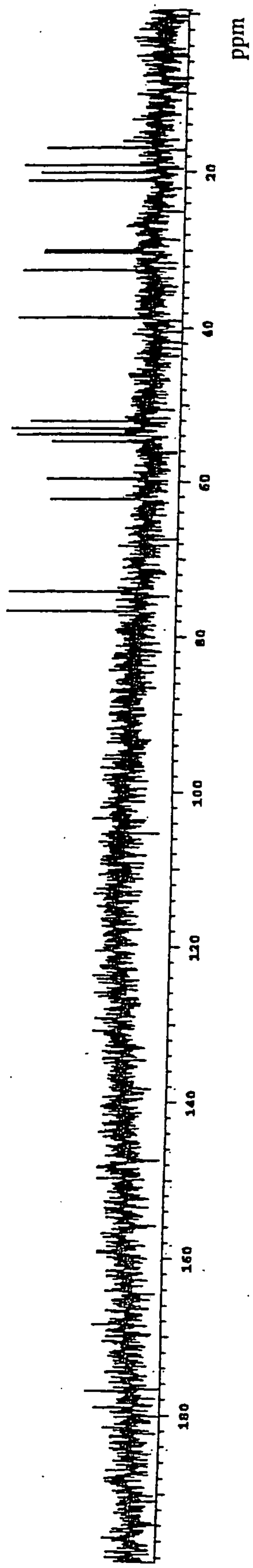

FIG. 5

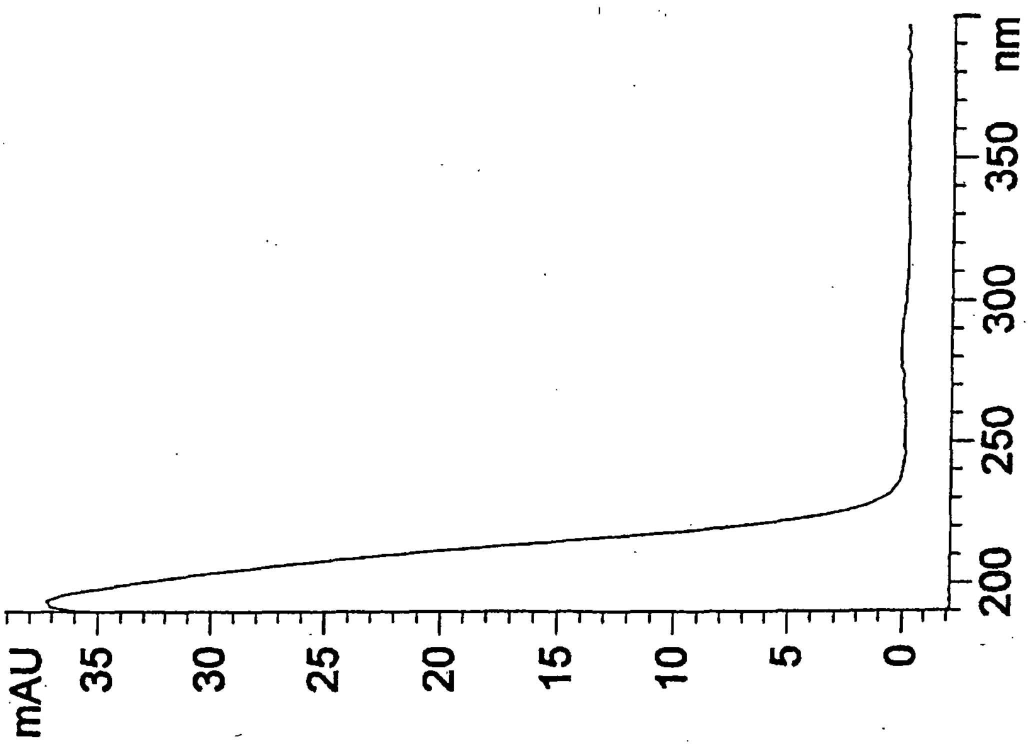

FIG. 6

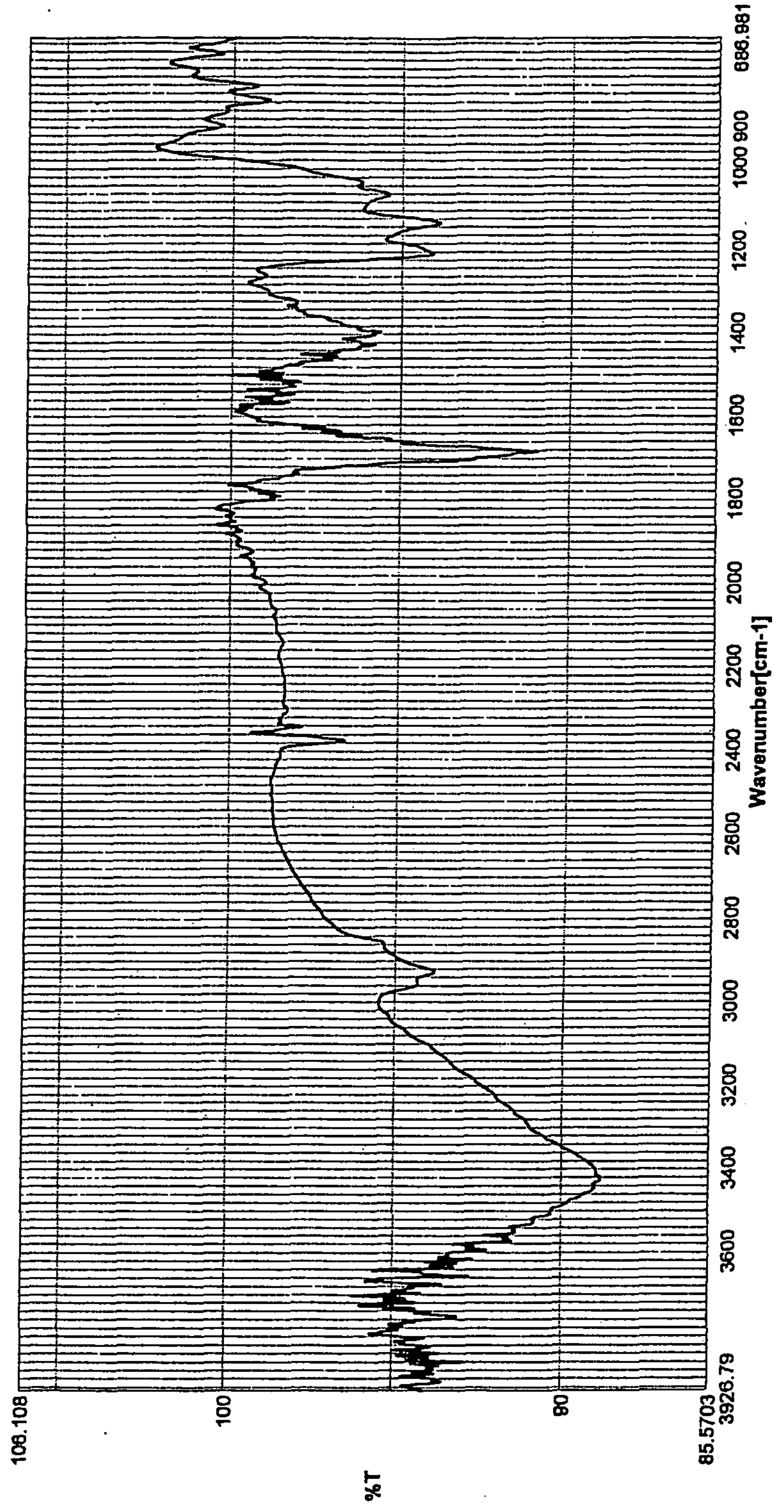
%T
108.108
100
90
85.5703
3926.79
3600
3400
3200
3000
2800
2600
2400
2200
2000
1800
1600
1400
1200
1000
900
686.981
Wavenumber[cm-1]

FIG. 7

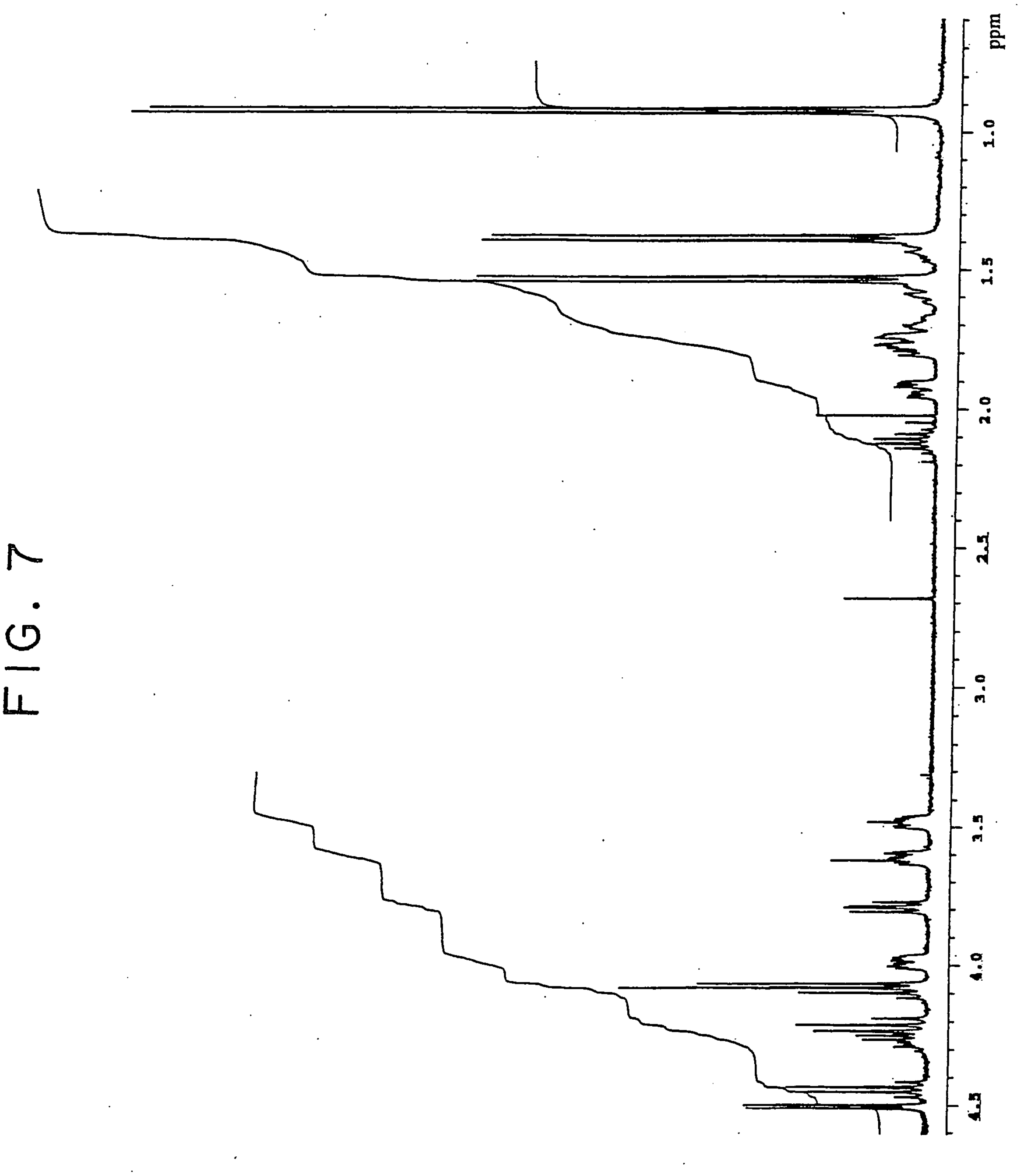

FIG. 8

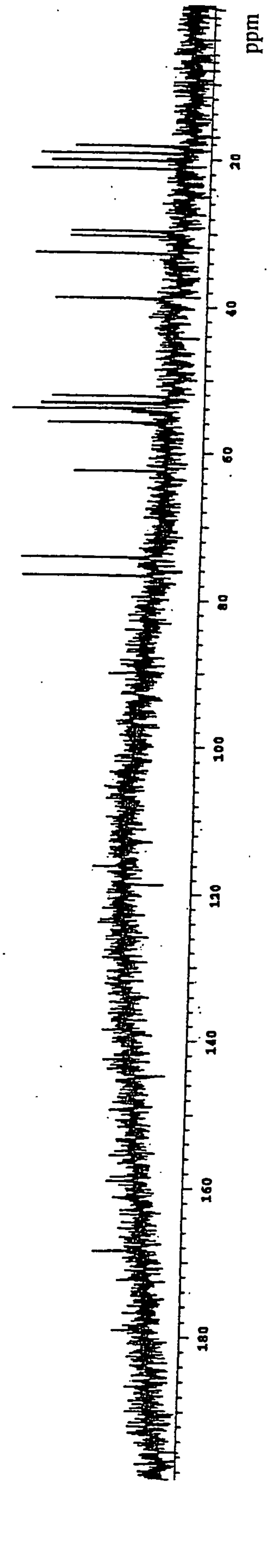

**INTERNATIONAL SEARCH REPORT**

International application No.
PCT/JP2004/001311

A. CLASSIFICATION OF SUBJECT MATTER
Int.Cl[7] C07K5/062, C12P21/02, C12N1/20//(C12P21/02, C12R1:465)
(C12N1/20, C12R1:465)

According to International Patent Classification (IPC) or to both national classification and IPC

B. FIELDS SEARCHED

Minimum documentation searched (classification system followed by classification symbols)
Int.Cl[7] C07K5/062, C12P17/00-17/18, 21/02

Documentation searched other than minimum documentation to the extent that such documents are included in the fields searched

Electronic data base consulted during the international search (name of data base and, where practicable, search terms used)
BIOSIS/WPI(DIALOG), CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | SHREDER K. et al., "Synthesis of a Constrained Enkephalin Analog to Illustrate a Novel Route to the Piperazinone Ring Structure.", Tetrahedron Lett., 1998, Vol.39, pages 221 to 224 | 1-11 |
| A | HUECK C.J. et al., "Type III Protein Secretion Systems in Bacterial Pathogens of Animals and Plants.", Microbiol.Mol.Biol.Rev. 1998, Vol.62, No.2, pages 379 to 433 | 1-11 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 February, 2004 (25.02.04) | 09 March, 2004 (09.03.04) |
| Name and mailing address of the ISA/ Japanese Patent Office | Authorized officer |
| Facsimile No. | Telephone No. |

Form PCT/ISA/210 (second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02057760 A1
- US 6586200 B


### Non-patent literature cited in the description


- *Microbiology and Molecular Biology Reviews,* June 1998, 381
- *Microbiology and Molecular Biology Reviews,* June 1998, 382 ff
- *J. Exp. Med.,* 16 November 1998, vol. 188 (10), 1907-1916
- *Infection and Immunity,* June 2000, 3689-3695
- **E. B SHIRLING ; D. GOTTLIEB.** *International Journal of Systematic Bacteriology,* 1966, vol. 16, 313
- Color Harmony Manual. 1958
- Bergey' s Manual of Systematic Bacteriology. 1989, vol. 4